# EUROPEAN PATENT SPECIFICATION

(11) **EP 0 813 442 B1**
(45) Date of publication and mention of the grant of the patent: **20.11.2002**
(21) Application number: 96910382.9
(22) Date of filing: 07.03.1996
(51) Int. Cl.: B01D 21/26, B01D 37/00, C12S 3/22, G01N 27/26, G01N 27/447, B01D 63/08

(54) **METHOD FOR ENRICHING RARE CELL POPULATION**
METHODE ZUR ERREICHERUNG VON POPULATIONEN SELTENER ZELLEN
PROCEDE POUR L'ENRICHISSEMENT DE POPULATIONS DE CELLULES RARES

(30) Priority: 08.03.1995 US 401131; 20.10.1995 WO PCT/US95/13631
(43) Date of publication of application: 29.12.1997
(73) Proprietor: BIOSEPARATIONS, INC., Tucson, AZ 85719 (US)
(72) Inventor: SAMMONS, David, W., Tucson, AZ 85718 (US); MANLEY, Michael, Rucson, AZ 85718 (US); UTERMOHLEN, Joseph, G., Tucson, AZ 85711 (US); TWITTY, Garland, E., Tucson, AZ 85715 (US)
(74) Representative: Marsh, Roy David
(86) International application number: US9603165
(87) International publication number: WO96027420

(56) References cited:
- WO-A-93/10442
- WO-A-94/17209
- US-A- 4 925 572
- US-A- 5 192 553
- US-A- 5 336 387
- US-A- 5 432 054
- US-A- 5 489 386

## Description

### Background of the Invention

The present invention relates generally to separation of rare cell populations from whole blood samples. More particularly, the present invention provides a non-invasive method for enriching the populations of rare cells, such as nucleated fetal erythrocytes or nucleated fetal red blood cells ("NRBCs") obtained from maternal blood samples by separating the NRBCs from the mother's erythrocytes, leukocytes and other blood components. Those skilled in the art will appreciate that the present method are not confined, in their utility, to enrichment of fetal NRBCs. Rather, a wide variety of rare cell populations present in the peripheral blood circulation may be separated according to the present method, as will be explained more fully herein.

In accordance with a preferred embodiment thereof, the present invention offers a method for enriching a population of fetal-origin NRBCs from a maternal blood sample by a negative selection process which concentrates the NRBCs by lysis and yields viable rare cell populations which may be cultured or otherwise analyzed. While enrichment of viable fetal-origin NRBCs is described herein as an operative model of the present method, the present invention is not so limited in its utility.

Physicians have long sought to develop non-invasive methods for prenatal diagnosis because the available methods, amnio-centesis and chorionic villus sampling (CVS) are potentially harmful to the mother and to the fetus. The rate of miscarriage for pregnant women undergoing amniocentesis is increased by 0.5 - 1%, and that figure is be slightly higher for CVS. Because of the inherent risks posed by amniocentesis and CVS, these procedures are offered primarily to older women, *i.e.*, those over 35 years of age, who have a statistically greater probability of bearing children with congenital defects.

Some non-invasive methods have already been developed to diagnose specific congenital defects. For example, maternal serum alpha-fetoprotein, and levels of unconjugated estriol and human chorionic gonadotropin can be used to identify a proportion of fetuses with Downs syndrome, however, it is not one hundred percent accurate. Similarly, ultrasonography is used to determine congenital defects involving neural tube defects and limb abnormalities, but it is useful only after fifteen weeks gestation.

The presence of fetal cells in the maternal peripheral circulation has been known for some time. However, fetal cells represent a rare cell population in maternal circulation present in estimated numbers ranging from 1 in 100,000 to 1 in 1,000,000. Fetal lymphocytes (Walknowska, *et al., Lancet 1*:1119 (1969), Schroder, *et al., Blood 39*:153 (1972; Schroder, *Scand J. Immunol. 4*:279 (1975)), syncytiotrophoblasts (Douglas *et al., Am. J. Obstet. Gynecol. 78*:960 (1959); Goodfells, *et al., Brit. J. Obstet. Gynecol. 89*:65 (1982); Covone, *et al., Lancet 2*:841 (984); Kozma, *et al., Hum. Reprod. 1*:335 (1986)), cytotrophoblasts (Mueller, *et al., Lancet 336:197* (1990)), and erythrocytes (Freese, *et al., Obstet. Gynecol.*, 22:527 (1963); Clayton, *et al., Obstet. Gynecol. 23*:915 (1964); Schroder, *J. Med. Genet. 12*:230 *(1975;* Medaris, *et al.*, *Am. J. Obstet. Gynecol. 148*:290 (1984)) have been identified in the maternal peripheral blood circulation.

Separation of nucleated fetal erythrocytes from maternal blood has been identified as a desirable method for facilitating prenatal diagnosis of genetic disorders. Fetal NRBCs have been separated from maternal blood by flow cytometry using a lysing reagent (European Published Patent Application No. 582736, published February 16, 1994); by triple gradient discontinuous gradient gel electrophoresis (Bhat, et al, U.S. Patent No. 5,275,933, issued January 4, 1994); by separation from nucleated cells using leukocyte depletion and ammonium chloride lysis of enucleated erythrocytes (Goldbard, PCT Publication WO 9417209, published August 4, 1994); by use of anti-CD71 monoclonal antibody and magnetic beads and in-situ fluorescence hybridization (FISH) (Ahlert, et al, German Published Patent Application No. 4222573, published August 12, 1993) or by other antibodies specific to a fetal erythrocyte antigen (Bianchi, PCT Publication WO 9107660, published May 30, 1991). Unfortunately, to date, there are no clinically acceptable methods for prenatal genetic diagnosis using maternal peripheral blood samples. Amniocentesis and CVS continue to be the only options available to a small percentage of pregnant women. These women represent the group statistically at risk of conceiving a fetus having genetic abnormalities.

Substantial attention has been given to methods of prenatal sex diagnosis using fetal cells in the maternal peripheral blood. Because obtaining a maternal blood sample is far less invasive than amniocentesis, CVS or fetal blood sampling, methods which are capable of enriching fetal cells from maternal peripheral blood samples have become a paramount focus of neonatology. Many studies have been made on prenatal sex diagnosis by means of the polymerase chain reaction (PCR) amplifying the Y-chromosome-specific DNA sequence in maternal blood. See, *e.g*., Bianchi, D.W., et al., *Proc. Natl. Acad. Sci. USA, 87*:3279-3283 (1990), Bianchi, D.W., et al. *Prenat. Diagn., 13*:293-300 (1993), Wachtel, S., et al., *Hum. Reprod., 6*:1466-1469 (1991), Suzemori, K., et al., *Obstet. Gynecol., 80*:150-154 (1992). In some of the studies using PCR amplification, fetal cells in the maternal blood were enriched and analyzed by PCR. While the sensitivities and specificities of the methods reported in the PCR studies is high, e.g., 64-100 and 80-100 percent, respectively, and the positive and negative predictive values were 75-100 and 67-100 percent, respectively, the diagnostic accuracy reported in each study was insufficient to be acceptable for routine clinical use. Hamada, H., et al., *Prenat. Diag., 15*:78-81 (1995).

Flow cytometry has also been used to separate fetal NRBCs on the basis of positive CD71 antibody (transferrin receptor) and glycophorin-A antibody binding. PCR conducted on the flow-sorted cells identified male fetuses at a rate of 100% accuracy and female fetuses at a rate of 83% accuracy. Simpson, J.L., et al., *J. Am. Med. Ass'n. 270:2357*-2361 (1993). Immunogenetic procedures have been combined with fluorescence-activated cell sorting (FACS) procedures to enrich fetal cells in whole blood from pregnant women. Herzenberg, L.A., et al., *Proc. Natl. Acad. Sci. USA, 76:*1453-1455 (1979) employed rabbit anti-HLA-A2 antibodies in conjunction with fluorescein-conjugated goat anti-rabbit immunoglobulins and analyzed the fluorescein-bound cells using FACS.

Fluorescence *in situ* hybridization (FISH) offers a method for identifying desired DNA in a cell at the interphase stage. FISH with conventional cytogenetic methods has been used on fetal cells recovered from maternal blood samples for sex determination, Wessman, M., et al., *Prenat. Diag., 12*:993-1000 (1992), and to detect chromosomal abnormalities, Simpson, J.L., et al., *Prenat. Diag., 12*:S12 (Supp. 1992) [fetal trisomy 21], Bianchi, D. et al, Prenatal Diagnosis through the Analysis of Fetal Cells in the Maternal Circulation, Genetic Disorders and the Fetus 3d Ed., Milunsky, A., ed., pp. 759-770 (1992) [Fetal trisomy 18].

Fetal progenitor cells have also been enriched from maternal blood by ligand binding onto an immobilization medium. CellPro, Inc., International Publication No. WO 94/25873, published November 10, 1994, discloses an immunoselection method for enriching fetal erythroid progenitor cells from maternal blood by separating a large fraction of maternal erythrocytes from the blood sample, such as by density gradient centrifugation on a Ficoll gradient or by preferential lysis of maternal erythrocytes in the presence of ammonium chloride, potassium chloride and a carbonic anhydrase inhibitor, then incubating a sample of maternal blood with a ligand to bind fetal progenitor cells and then removing unbound blood products, leaving the enriched, ligand-bound fetal erythroid progenitor cells. The ligands disclosed include antibodies, erythropoietin or transferrin. The ligand is immobilized on any of a variety of solid supports, such as hollow fibers, magnetic beads, plates, dishes, flasks, meshes, screens, solid fibers, membranes or dipsticks.

The CellPro International Publication, discloses, for example, the use of first member-second member binding pairs, including biotin-avidin, biotin-streptavidin, biocytin-avidin, biocytin-streptavidin, methotrexate-dihydrofolate reductase, 5-fluorouracil-thymidylate synthetase and riboflavin-riboflavin binding protein, wherein the first binding member is linked to a ligand capable of binding fetal nucleated erythroid cells, the second binding member is linked to an immobilization medium, and the second member has a binding affinity constant for the second member of greater than about 10⁸M⁻¹. The preferred embodiments disclosed include a methods for positive and negative selection of fetal cells, useful independently or in conjunction with one another. The positive selection process using a binding pair consisting of biotinylated anti-CD34 antibody and immobilized avidin. The CD34 antigen is expressed on fetal progenitor cells and hematopoietic progenitor cells, however, in normal adults, the hematopoietic progenitor cells reside in the bone marrow and CD34 positive cells are found in the peripheral circulation at a rate of less than 0.1 %. The negative selection process using a binding pair consisting of biotinylated anti-CD45 and immobilized avidin. The CD45 antigen is expressed on maternal erythrocytes, but not fetal progenitor cells. The immunoselected fetal cells then may be subjected to analysis by karyotyping, PCR, RFLP, SSCP or FISH to provide genetic or biochemical information. The CellPro method, however, suffers from a clinically unacceptable yield of enriched cells. While this published application states enrichment of fetal progenitor cells to a concentration greater than 1%, the highest level of enrichment supported by the examples was attained by a dual positive selection process using sequential CD34 antibody binding steps yielding a sample containing about 1 in 2,000 or 0.5% fetal cells, representing about a 500-fold enrichment from the starting sample.

To date, however, no clinically acceptable method for enrichment of rare cell populations, particularly fetal nucleated erythrocytes, from peripheral blood samples has been devised which yields cell populations sufficient to permit clinical diagnosis. The clinical need for a method capable of producing higher yields of rare fetal cell populations separated from maternal whole blood was recently underscored by Hamada, H., *et al., Prenat. Diag. 15:*78-81 (1995) who stated that "The data obtained in this study suggest that fetal sex determination using maternal peripheral blood with FISH is possible and that this diagnostic method will be clinically useful when more cells are analyzed."

### Summary of the Invention

The present invention provides a method for separation of viable, culturable fetal nucleated red blood cells from maternal whole blood constituents at clinically useful levels. The enriched fetal NRBCs are present in numbers sufficiently large for effective cell culture and subsequent genetic diagnosis with conventional and cytogenetic methods, as well as emerging DNA methods such as PCR, RFLP, SSCP, FISH chromosome banding and karyotyping.

Accordingly, the present invention provide a method for recovering a desired cell population selected from the group consisting of nucleated cells, erythroid progenitor cells and fetal nucleated erythrocytes from a whole blood sample, comprising the steps of:
a) depleting a major fraction of unwanted cells from the whole blood sample, thereby concentrating a desired cell population;
b) passing the concentrated desired cell population through a counterflow stabilized charge-flow separator apparatus, in which the mobility of each cellular component in the blood sample in the presence of an applied electrical field is opposed by a buffer counterflow, thereby concentrating the desired cell population from other cellular blood components;
c) retrieving at least one fraction eluting from the charge-flow separator apparatus having a concentrated desired cell population; and
d) recovering an enriched desired cell population for analysis.

In a preferred embodiment the present invention achieves its objectives by a) density gradient centrifugation of whole blood to provide a gross separation of a major fraction of maternal erythrocytes and blood plasma from whole blood, b) charged-flow separation of the gross separated whole blood to separate fetal erythroid progenitor cells from maternal myeloid and lymphoid cells, c) ammonium lysis of remaining maternal erythrocytes and d) complement mediated lysis to remove myeloid and lymphoid cells present in the charged flow separated fractions. The inventive system and method achieves enrichment of rare cell populations, fetal erythroid progenitor cells, specifically, CFU-GEMM (colony forming unit, granulocyte, erythroid, megakaryocyte, monocyte), BFU-E (burst-forming unit, erythroid), CFU-E (colony-forming unit, erythroid), pronormoblasts, normoblasts (erythoblasts), and reticulocytes by negative selection for the desired cell population.

The charged-flow separation (CFS) apparatus described in US 5 662 813 (pub. 2.9.97), respectively in WO 96 12945 (pub. 2.5.96) or as described in our U.S. Patents Nos. 5,336,387 and 5,173,164, which are also hereby incorporated by reference, is ideally suited for separation rare cell populations from whole blood. The CFS apparatus is preferably run under sterile conditions to yield culturable separated cells. Under charge-flow separation conditions, as described in the Twitty, et al. application, or as described in U.S. Patents Nos. 5,336,387 or 5,173,164, fetal erythroid progenitor cells exhibit consistent migration patterns in an electric field according to surface charge density which are different and distinct from the migration patterns of mature enucleated erythrocytes. By using the CFS apparatus coupled with the pre- and post-processing methods described more fully hereinafter, the present invention has achieved enrichment of up to 12 percent of rare cell populations in the final sample.

The CFS system and method has been successfully used to recover nucleated red blood cells from the peripheral blood circulation of pregnant women. The recovered NRBCs were identified histologically. The NRBCs exhibited consistent migration patterns whether they came from maternal blood or from umbilical cord blood collected at birth. No NRBCs were found in blood from nulliparous women.

Because the inventive system and method of charge-flow separation of NRBCs from maternal blood is based on the intrinsic physical properties of the NRBCs, there is little need for extensive preparation of the maternal blood sample with antibodies or ligands. The cells may be processed at greater than or equal to 60,000 cells per second and specialized training is not required. When the inventive charge-flow separation system and method is used, the recovered cells are viable, thus raising the possibility of further enrichment by cell culture.

In conjunction with or in addition to the charge-flow separation system and method, the present invention also includes an affinity separation method for separating NRBCs from other cell populations in a maternal blood sample. The adsorption-filtration affinity method of the present invention entails layering a maternal blood sample onto a fibrous adsorption-filtration filter medium having a nominal pore size of about 8 microns and being capable of 40-80% leukocyte immobilization, with a 70-80% post-wash leukocyte retention rate, and which is extremely hydrophilic, being capable of wetting with solutions having surface tensions of up to 85-90 dynes, which has a hold up volume of 40-70µl/cm² for a single layer of adsorption-filtration filter medium and which is characterized by low to medium protein binding. The preferred adsorption-filtration separation filter medium is that sold by Pall Corporation under the trademarks "LEUKOSORB" TYPES A and B or that described in U.S. Patent Nos. 4,923,620, 4,925,572, or European Patent No. 313348.

The charge-flow separation system and method and the adsorption-filtration separation system and method of the present invention may be used separately or may be used in conjunction with one another to achieve enrichment of the nucleated fetal red blood cell population in a maternal blood sample.

### Brief Description of the Figures

Fig. 1 is a flow diagram of representing hemopoiesis and differentiation between erythroid, myeloid and lymphoid cell lines.
Fig. 2 is a flow diagram of the inventive enrichment process for separating rare cell populations from whole blood.

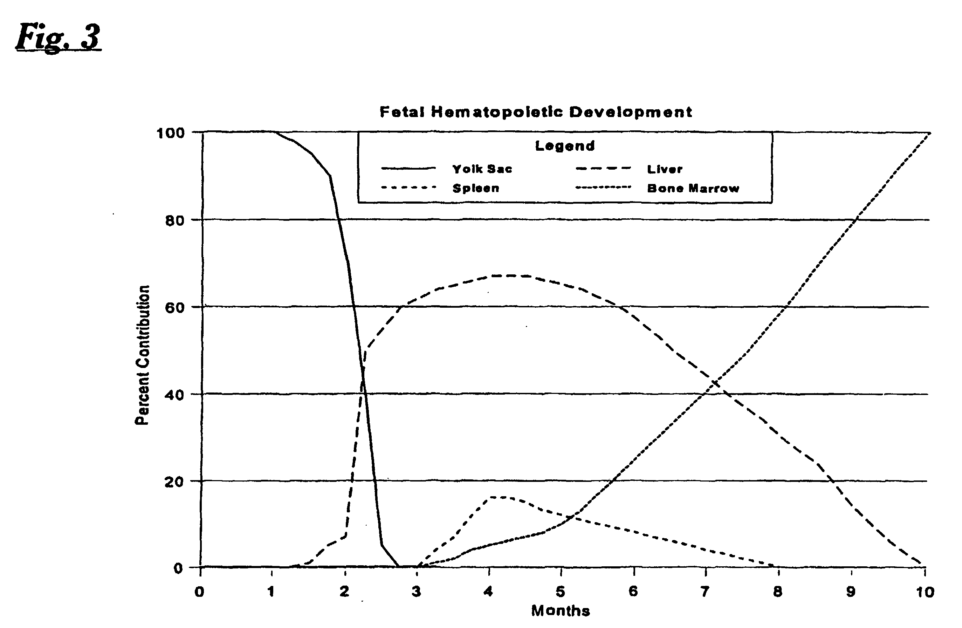

Fig. 3 is a graph representing fetal hematopoietic development and sites during gestation.
Fig. 4. is a karyotype image of a 51 year old male obtained from cultured erythroid progenitor cells isolated from a whole blood sample using the method of the present invention.
Fig. 5 is a flow diagram illustrating a first embodiment of the adjunct adsorption-filtration supports in accordance with of the present invention.
Fig. 6 is a flow diagram illustrating a second embodiment of the adjunct adsorption-filtration supports in accordance with of the present invention.
Fig. 7 is a flow diagram illustrating a third embodiment of the adjunct adsorption-filtration supports in accordance with of the present invention.
Fig. 8 is a flow diagram illustrating a fourth embodiment of the adsorption-filtration supports used as an adjunct to the enrichment method of the present invention.

### Description of Preferred Embodiments

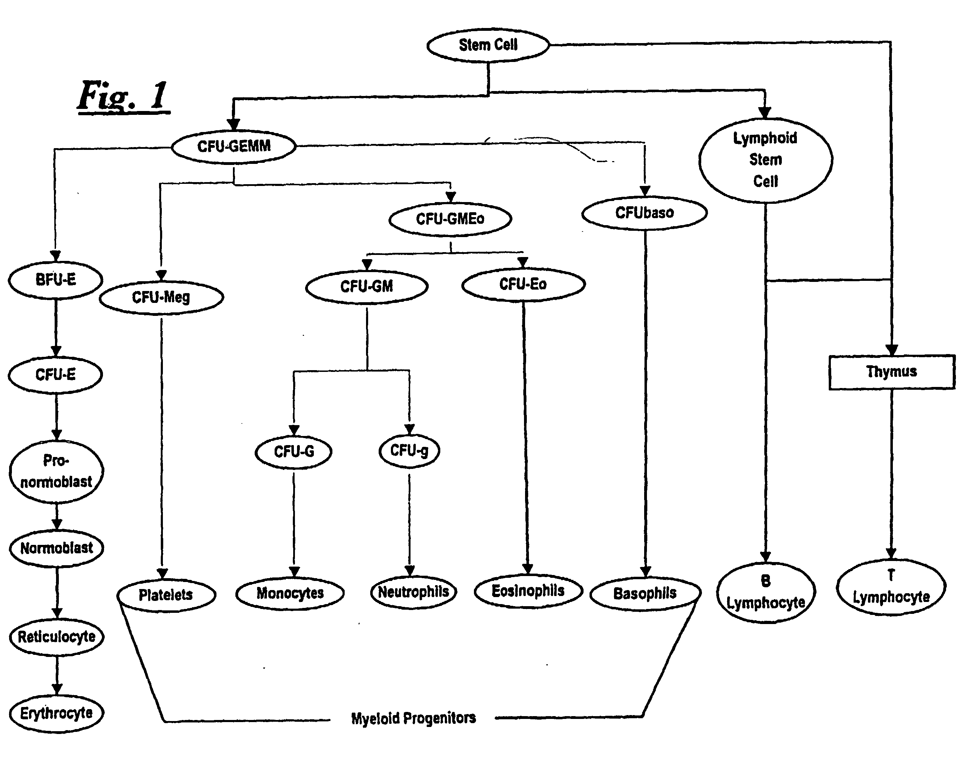

Fig. 1 is a diagram depicting the hematopoietic system and progenitor hierarchy starting from a common pool of pluripotent hematopoietic stem cells, through commitment to erythroid, myeloid and lymphoid cell lines, to unique morphology cell lines. As those skilled in the art will understand, the process of mature blood cell production from stem cells is a complex series of irreversible cellular differentiation and amplifying proliferation events. The hematopoietic system provides the backdrop against which the system and method for separating rare cell populations from whole blood may be viewed.

It is well known in the art that as stem cells differentiate during hematopoiesis, the maturing cells undergo morphological changes. Concomitant with these morphological changes, the cells develop different sets of surface molecules, i.e., proteins, polysaccharides, etc., such as those exemplified by the cluster differentiation (CD) series to classify the surface molecules. Antibodies specific for different surface molecules, such as the CD series, may then be used to identify the presence of specific cell populations in a blood sample. It has been found, however, that various erythroid, myeloid and lymphoid cells share some common CD surface molecules, as do individual cell types within each development family, e.g., erythropoietic cells. These shared CD surface molecules make selection processes cumbersome. A good summary of principal features of known CD molecules present in hematopoietic cell lines is found in Hoffbrand, A.V., *et al.*, Essential Haematology, 3d ed., Blackwell Scientific Publications, pp. 417-418, (1993).

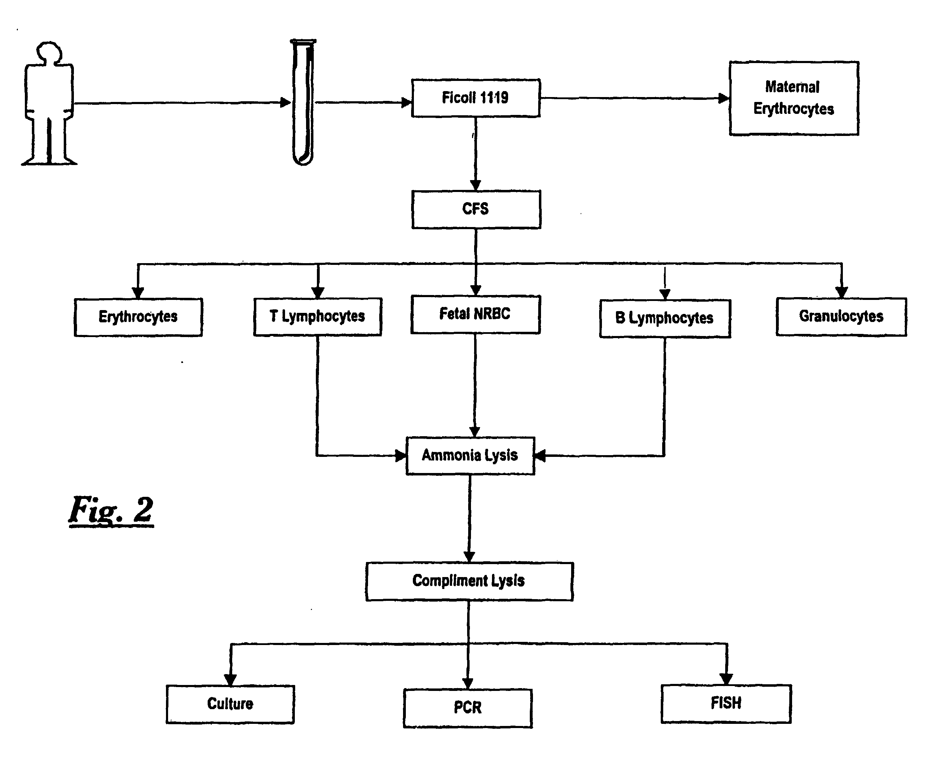

In accordance with a first preferred embodiment of the present invention, and with particular reference to Figure 2, there is provided a method for enriching rare cell populations from whole blood. It is axiomatic that all healthy human blood samples, whether male, female, child or adult, young or aged, irrespective of blood type and Rh factor, is constituted of a full range of blood cell types along the hematopoietic hierarchy depicted in Figure 1. When a person is afflicted with a particular cellular disorder, such as breast cancer, prostate cancer, lung cancer, leukemia, or the like, abnormal cells representative of the disorder are usually found, in varying degrees, as rare populations in the peripheral blood circulation. Similarly, it is well known that fetal-origin cells may be found at very low levels, i.e., 1 x 10⁻⁶ to 1 x 10^{-7.} in the maternal peripheral blood circulation. By using alternating negative selection steps (defined herein as selecting the undesired cell populations by any means, including antibody or ligand binding, filtration, or manual selection) and positive selection steps (defined herein as selecting the desired cell populations, including antibody or ligand binding, filtration, or manual selection), the method of the present invention isolates rare cells from peripheral blood, by enriching the desired rare cell population from whole blood.

As depicted in Figure 2, a peripheral blood sample is obtained from a subject, by any conventional technique known in the art, such as by venipuncture, usually of the antecubital vein. The volume of blood collected typically ranges between 20-40 ml, but may be more or less. The blood is collected in vacuum containers, typically in the presence of one or more anticoagulants, as desired, including acid-citrate-dextrose (ACD), ethylenediaminetetraacetic acid (EDTA), heparin, and citrate-phosphate-dextrose-adenine (CPDA). The collected blood sample may be stored for up to 4 days at 4°C. It is preferable, however, to process and analyze the blood sample within 24 hours of collection It has been found that after 24 hours, the blood sample will undergo changes, such as maturation of some hemotopoietic cells present in the blood sample.

The whole blood sample is layered onto Ficoll/Hypaque-1119 (polysucrose type 400/sodium diatrizoate, 1.119 g/ml) (HISTOPAQUE-1119, Sigma Chemical Company) density gradient and centrifuged at about 400 X G for about 40 minutes. After centrifugation, plasma forms a top layer, a white band of nucleated cells, including nucleated erythroid cells and lymphocytes, is immediately below the plasma layer, the Ficoll/Hypaque-1119 containing a broad diffuse band containing other nucleated erythroid cells and immature red cells with densities heavier than the white layer but lighter than the mature erythrocyte pellet is immediately below the nucleated cells and the erythrocyte pelle is present at the very bottom of the tube.

The plasma fraction is discarded and the nuclear fraction is removed by decanting or aspiration without disturbing the nuclear fraction. The nucleated cell portion is removed to a separate sterile centrifuge tube, washed in phosphate buffered saline and prepared for loading onto the charged flow separator apparatus and subjected to charged flow separation as more fully disclosed in the Twitty, et al. patent application and U.S. Patent Nos. 5,336,387 and 5,173,164, under conditions to maintain a sterile environment.

It has been found that the mobility of fetal NRBCs in an electric field is intermediate between the fastest enucleated red blood cells and the slowest white blood cells. Thus, for example, where enucleated red blood cells elute in fractions 4-7, and leukocytes elute from fractions 6-8, it has been found that the nucleated fetal red blood cells elute in fractions 5-8 with the peak in fraction 7. Based upon these findings, it has been determined that fetal NRBCs have electrophoretic mobilities more similar to many mononuclear cells of lymphoid origin such as T lymphocytes and B lymphocytes, than to erythrocytes or a majority of granulocytes or other cells of myeloid origin. It is desirable, therefore, to collect not only the peak fraction, but also the two fractions adjacent the peak fraction, e.g., fractions 6 and 8. In this manner, the nuclear fraction eluting from the CFS apparatus is separated from a major fraction of mature erythrocytes, granulocytes and other cells of myeloid origin in the original blood sample.

To test the suitability of CFS-separation as a clinically acceptable separation tool for providing cells for clinical diagnosis, sex determination, fluorescence *in situ* hybridization (FISH) was performed on the CFS-separated nucleated cell portion. FISH analysis confirmed that the CFS enrichment method, as described above, obtains enrichment levels sufficient to make clinically reliable diagnosis. Ficoll/Hypaque-1119 density gradient centrifugation, followed only by CFS-separation, yielded fetal NRBC frequencies in the range of 1/20 to 1/50, or about 2-5% fetal NRBCs. Table 1, below, summarizes data from blood samples obtained from five pregnant women at 20-30 weeks gestation, together with blood samples from a nulliparous female control and a male control. All samples were processed as described above and analyzed by FISH using a DYZ1/DYZ3 probe specific for the Y chromosome.

**Table 1**

| **Case** | **Gestation (Weeks)** | **Fetal Sex** | **Nuclei Labeled/ Nuclei Counted** | **Percent Labeled** |
|---|---|---|---|---|
| 1 | NR | Male | 15/1000 | 1.5 |
| 2 | 20.5 | Male | 15/1000 | 1.5 |
| 3 | 30 | Male | 46/2500 | 1.8 |
| 4 | 20 | Male | 13/1000 | 1.3 |
| 5 | 23 | Male | 29/1000 | 2.9 |
| Male Control | | | 988/1000 | 98.8 |
| Female Control | | | 3/1000 | 0.3 |

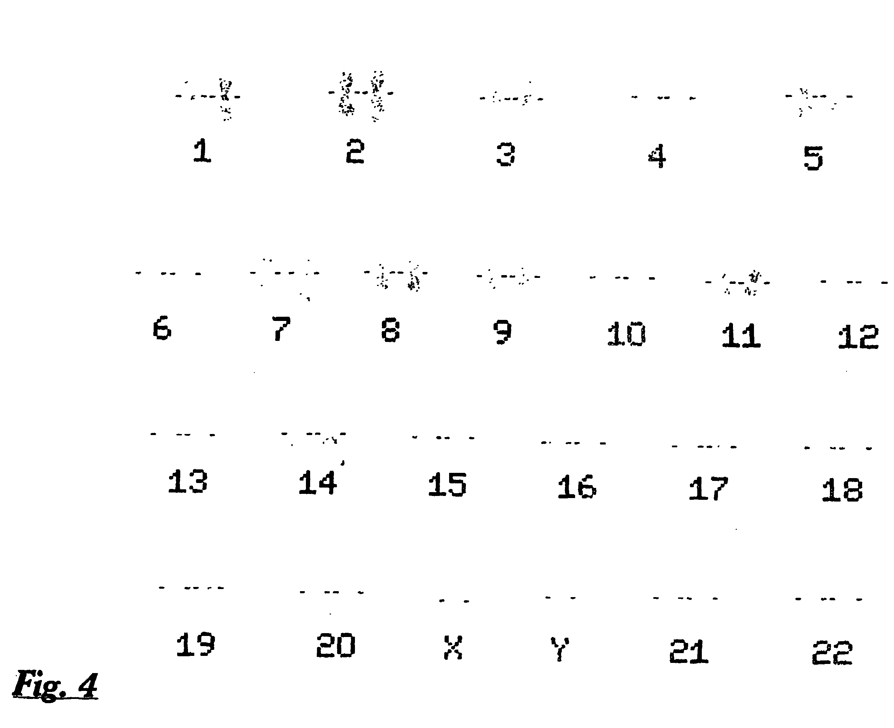

The suitability of the CFS-enriched fetal NRBCs for clinical prenatal genetic diagnosis was determined by processing whole blood samples from one woman taken two days post-partum with a stillborn delivery and two pregnant women who had previously undergone amniocentesis at 16 and 19 weeks, respectively, with known results. Karyotyping of the still-born fetal blood sample confirmed a positive fetal trisomy 18 with karyotype 47,XX,+18, while amniocentesis on second subject indicated a fetal karyotype 47,XX, +21 and third subject indicated a fetal karyotype 471XY,+21+m, both confirming positive fetal trisomy 21. Blood samples from normal adult males were used as controls. An example of a karyotype from a normal adult male is presented as Figure 4.

The subjects' blood samples and the controls were processed by Ficoll-1119 gradient centrifugation followed by CFS-separation as described above. Following charged flow separation, the trisomy 18 positive sample and the first male control sample were analyzed by FISH using a chromosome-18-specific probe, and the trisomy 21 positive samples and the second male control sample were analyzed using a chromosome-21-specific probe. The data for the known fetal trisomy 18 sample is presented below in Table 2 and the data for the known fetal trisomy 21 sample is presented below in Table 3.

**Table 2**

| | **Percent nuclei containing *n* signals** | | | | |
|---|---|---|---|---|---|
| **Sample** | ***n*=0** | **1** | **2** | **3** | **≥ 4** |
| Trisomy 18 | 3 | 26 | 70 | 2 | 0 |
| Control A | 0 | 5 | 95 | 0 | 0 |
| Control B | 0 | 21 | 79 | 0 | 0 |

**Table 3**

| | **No. Cells Scored** | **Percent nuclei containing *n* signals** | | | | |
|---|---|---|---|---|---|---|
| **Sample** | | ***n*=0** | **1** | **2** | **3** | **≥4** |
| Case 1 | 1000 | 6 | 16 | 69 | 7 | 2 |
| Case 2 | | | | | | |
| Sample 2(a) | 1000 | 11 | 23 | 61 | 5 | 0.4 |
| Sample 2(b) | 200 | 0 | 1 | 93 | 4 | 2 |
| Sample 2(c) | 500 | 26 | 36 | 36 | 2 | 0 |
| Control | 500 | 3 | 24 | 73 | 0.4 | 0 |

Table 4, below, summarizes the results of genetic detection of CFS-processed fetal cells analyzed by FISH in accordance with the FISH protocol published and distributed by Oncor, Inc. As of the date of this application for use with the Oncor, Inc. FISH kit. .

**Table 4**

| Probe | No. Samples | Sample Type | Mean % Cells Labeled ( SD) |
|---|---|---|---|
| DYZ1/DYZ3 (Y Chromosome) | 5 | Experimental | 1.8 (0.6) |
| | 2 | Male Control | 98.8 (0.8) |
| | 2 | Female Control | 0.3 |
| D18Z1 (Chromosome 18) | 1 | Experimental | 2.0 |
| | 2 | Male Control | 0 |
| D21S1219/ D21S1220 (Chromosome 21) | 2 | Experimental | 5.3 (2.4) |
| | 1 | Female Control | 0.4 |

Because the relative mobilities of lymphocytes and fetal NRBCs are similar, it was deemed desirable to further enrich the fetal NRBC population obtained by charge flow separation. The charge flow separated sample was then further enriched by negative selection of the remaining erythrocytes and myeloid cell populations.

The enriched nucleated cell fraction eluting from the CFS apparatus typically contains a small fraction of mature erythrocytes, a major fraction of mononuclear lymphoid cells, such as B and T lymphocytes, a minor fraction of fetal NRBCs and a minor fraction of myeloid-lineage cells.

Ammonia hemolysis preferentially eliminates mature erythrocytes from the enriched nucleated CFS fetal cell eluate. The rate controlling factor in ammonium hemolysis is the level of erythrocytic carbonic anhydrase present in the cell. Fetal erythroid cells have carbonic anhydrase levels one-fifth those of adult erythroid cells. As a result of this difference in carbonic anhydrase levels, selective ammonium hemolysis of mature erythroid cells is controlled as a time-rate function, i.e., for any given time period, adult red cells will lyse five times faster than fetal cells.

The enriched nuclear CFS eluate is incubated in the presence of ammonia or ammonia salts, for example ammonia chloride, chloride ions, for example sodium chloride, potassium chloride, magnesium chloride or calcium chloride, and a carbonic anhydrase inhibitor, such as acetazolamide, cyanide, cyanate, monovalent sulfides or sulfonamides, under conditions and for a time sufficient to allow ammonium ion transport within the cells, as described in Jacobs and Stewart, *J. Gen. Physiol. 25:539-552 (1942), or Maren and Wiley, Molec. Pharmacol. 6:430-440 (1970)*, followed by incubation in the presence of ammonia and carbon dioxide, to preferentially lyse adult erythrocytes in the sample.

The lysed erythrocytes are separated by centrifugation and the pellet is resuspended in PBS. The resulting product is now substantially free of maternal erythrocytes, but contains fetal nucleated erythroid cells and maternal myeloid and lymphoid cells. To remove a substantial number of maternal myeloid and lymphoid cells remaining in the sample, compliment lysis of the myeloid and lymphoid cells is undertaken. For example, anti-human CD37 IgM is added to the PBS suspension and allowed to bind to human lymphocytes present in the sample. After binding, rabbit complement is added and the resulting samples are allowed to incubate. Following incubation, incomplete Iscove's medium with only fetal calf serum (PCS) is added to the sample and the sample is centrifuged. The resulting pellets are may be prepared for culture PCR, FISH or other analytical method as desired in accordance with analytical protocols well known in the art. It is desirable to resuspend the pellet in Iscove's medium with only FCS and remove and treat aliquots using 3% acetic acid and crystal violet to lyse erythrocytes and stain nuclei. Nucleated cell counts of the aliquots may then be made under light microscopy.

In accordance with a further preferred embodiment of the invention, the resuspended cells are prepared for cell culture by diluting the cells with incomplete Iscove's medium in the presence of FCS so that each 0.1 mL contains 4 X 10⁵ nucleated cells. 0.1 mL of incomplete Iscove's medium containing 4 X 10⁵ nucleated cells is then added to 1.0 mL of complete Iscove's medium (containing growth factors and methylcellulose), which is then added to a 35mm Petri dish and cultured according to standard culture protocols as described in *Colony Assays of Hematopoietic Cells Using Methylcellulose Media*. An Introductory Technical Manual, Terry Fox Laboratory Media Preparation Service, Vancouver, B.C., Canada, 1992 ed.

The plates are scored for colonies at day 10-12 and again at day 18-21. At day 10-12, CFU-E and mature BFU-E colonies are noticeable, and at day 18-21, primitive BFU-E, CFU-GM and CFU-GEMM colonies are present and may be scored. At any desired point in colony growth, so long as mitotic cells are present, growth is synchronized via 5-fluorouracil block and thymidine release of the 5-fluorouracil block. See, e.g., Fraser, et al., *Cancer Genetics, Cytogenetics, 24:*1-6 (1987), hereby incorporated by reference for the 5-fluorouracil block procedures. Cellular growth is subsequently arrested in the metaphase portion of mitosis, using colecemid colcemid, colchicine, or similar substance, so that high mitotic indices may be obtained and karyotypes prepared. Growth synchronization is an optional, but preferred stem and usually yields higher mitotic indices.

### EXAMPLE

40 mL of whole blood was collected from a pregnant female by venipuncture. The blood sample was anticoagulated with CPD (2.55g d-glucose, 2.63g sodium citrate, 0.327g citric acid and 0.222 monobasic sodium phosphate in 100 mL distilled water) and placed in a 50 mL sterile centrifuge tube. A 50-100 µl aliquot was removed to take erythrocyte and lymphocyte counts. HISTOPAUQUE-1119 (Ficoll/Hypaque-1119, polysucrose type 400/sodium diatriazote, 1.119 g/ml, Sigma Chemical Company, Catalog Number 1119-1) was warmed to 37°C in a water bath. The blood may be held at room temperature or in the water bath at 37°C.

20 mL of HISTOPAQUE-1119 was aseptically placed into each of two sterile 50 mL centrifuge tubes and 20 mL of blood is carefully layered on top of the HISTOPAQUE-1119 in each tube. If there is an insufficient blood volume available to yield two 20 mL fractions, sterile phosphate buffered saline may be used to bring the blood volume in each tube to 20 mL. The blood-HISTOPAUQUE layered tubes are centrifuged at 430 X G in a sorwall HS-4 swing bucket rotor for 40 minutes at room temperature. After centrifugation, the upper plasma layer in each tube was decanted then discarded. The white nucleated cell band immediately above the HISTOPAQUE-1119 along with the diffuse erythroid band in the HISTOPAQUE-1119 were removed with a pipette and combined in a separate sterile 50 mL centrifuge tube. The mature erythrocyte pellet was discarded.

Sterile PBS at 37°C was added to the nuclear fraction pipetted to a separate sterile 50 mL centrifuge tube to a volume of 50 mL, and centrifuged at 1750 RPM for 20 minutes. The supernatant was decanted and the resulting pellet resuspended in 50 mL sterile PBS and centrifuged at 1500 RPM for 20 minutes. The supernatant was decanted and the pellet resuspended in 5 mL of buffer (triethanolamine, 5 mM; glycine, 280 mM, glucose, 22 mM, acetic acid to pH 7.3). The re-suspended cells were processed the a CFS apparatus configured with 12 separation channels, 10 counterflow input channels and 4 counterflow output channels. 5 mL of the cells resuspended in buffer were introduced in a sample input flow, with the sample buffer input having a flow rate of 0.220 ml/min/channel, with a buffer output flow rate of 0.370 ml/min/channel and a counterflow pump rate of 4.0 ml/min, in the presence of an applied electric field of 325 V at 50-55 mA.

After the CFS run, 12 fractions were collected in 50 mL centrifuge tubes. Fractions 4-6 were determined to contain cells and the fractions collected in centrifuge tubes 4-6 were centrifuged at 1750 RPM for 20 minutes, the supernatant was decanted and the resulting pellet was resuspended in 5-10 mL of sterile PBS and recentriguged at 1500 RPM for 10 minutes.

The supernatant was decanted and 10 mL of ammonium lysate (1 mL 10⁻³ acetazolamide, 9 mL NaCl and 90 mL of 0.1844 NH₄Cl) was added to each tube and incubated at room temperature for 3 minutes. After incubation, 2 mL of a 3mM solution of NH₄HCO₃ was added to each tube and incubated for an additional 4 minutes at room temperature. The tubes were then centrifuged at 1500 RPM for 10 minutes and the supernatant decanted. The pellets were resuspended in 0.9 mL of sterile PBS and 1 up of anti-human CD37 IgM (Research Diagnostics) was added to each tube and each tube was agitated until antibody was completely disbursed. After the antibody is dispersed, 100 ul of Low Tox Rabbit Complement for human lymphocytes (Accurate Antibody, Cedar Lane Laboratories, Hornby, Ontario, Canada) was added to each tube and incubated at 37°C for 30 minutes.

Following incubation with antibody and complement, 12 mL of incomplete Iscove's medium with 2% FCS (Stem Cell Technologies, Vancouver, B.C., Canada) was added to each tube and the tubes centrifuged at 1500 RPM for 10 minutes. The supernatant was removed and the pellets were resuspended in 0.5-5 mL of incomplete Iscove's medium with 2% FCS. Aliquots were removed and fixed with 3% acetic acid and crystal violet to lyse erythrocytes and stain nuclei. Nucleated cell counts were made on each aliquot.

The resuspended cells were then re-diluted with incomplete Iscove's medium with 2% FCS to a concentration of 4 x 10⁵ nucleated cells per 0.1 mL. 0.1 mL of re-diluted cells was added to 1.0 mL of complete Iscove's medium, containing growth factors and methyl cellulose), and this amount, 1.1 mL, was added to a 35 mm Petri dish and placed into culture in accordance with *Colony Assays of Hematopoietic Cells Using Methylcellulose Media. An Introductory Technical Manual,* Terry Fox Laboratory Media Preparation Service, Vancouver, B.C., Canada, 1992 ed.

The plates were scored for colonies at day 10 and again at day 18. At day 10, CFU-E and mature BFU-E colonies were present and at day 18, primitive BFU-E, CFU-GM and CFU-GEMM colonies were present.

On day 18, 50 µl of colcemid (Gibco, diluted 1/10) were evenly added to the culture dishes using an automatic pipette. The colcemid-added culture dishes were incubated for 45-60 minutes at 37°C in 5% co₂. 100 µl of hypotonic solution were added to several microfuge tubes. Colonies were pipetted from the culture dishes and individual colonies were mixed in each microfuge tube containing hypotonic solution and incubated for 20 minutes. The contents of each microfuge tube were then placed on a poly-1-lysine coated slide and incubated for 5 minutes.

20 ul of 20% fixative (3:1 methanol/acetic acid) was then added to each slide. The slides were then immersed in two changes of 5 minutes each of full strength fixative, followed by drying at 37°C overnight.

The cultured cells were then analyzed by FISH using the Y-chromosome probes DYZ1/DYZ3 (Oncor, Inc.) in accordance with the FISH protocol published and distributed as of the date of this application by Oncor, Inc. for use with the Oncor, Inc. FISH kit. The results of FISH analysis are detailed in Table 5, below:

**Table 5**

| **Slide No.** | **Colony Type** | **Slide Date** | **Y Signal** | **Metaphase (No. Cells)** | **Mitotic Index (%)** | **Total Cells** |
|---|---|---|---|---|---|---|
| CS1/P1/T1 | Red | 1/30/95 | 0 | 0 | 0.00 | 158 |
| CS1/P1/T1 | Myeloid | 1/30/95 | 5 | 1 | 0.21 | 476 |
| | | | | | | |
| CS2/P1/T1 | Mixed | 1/30/95 | 0 | 3 | 0.78 | 383 |
| CS2/P2/T1 | Red | 1/30/95 | 0 | 3 | 2.8 | 107 |
| | | | | | | |
| CS3/P1/T2 | Myeloid | 1/30/95 | 2 | 1 | 0.26 | 380 |
| CS3/P1/T3 | Mixed | 1/30/95 | 0 | 17 | 5.25 | 324 |
| CS3/P2/T2 | Red | 1/30/95 | 0 | 4 | 0.52 | 644 |
| Control | | | | | | |
| CFS100/F6 | Cytospin | 11/29/94 | 495 | | 98.61 | 502 |

Sister cells obtained from one subject (CS3) to those cultured and analyzed by FISH, as summarized in Table 5, above, were enriched and cultured in accordance with the above-described protocol. Twelve colonies were harvested on day 18 of culture from the sister cell line. PCR testing for Y-sequences in the CFS-eluates was conducted with SRY2 and SRY3 primers, each consisting of a 258 base pair sequence in SRY conserved motif. PCR was conducted in accordance with Perkin-Elmer's published PCT protocol, hereby incorporated by reference. Lanes 3 and 19 contained a 123 base pair marker. The presence of SRY-positive fetal cells in two colonies, 6 and 8, of the twelve colonies was confirmed by PCR. The presence of two positive colonies in twelve total colonies represents an enrichment level of 1 in 6 or 16.67%. The results of the PCR are summarized in Table 6, below:

**Table 6**

| **Lane** | **Sample ID** | **SRY Band** | **Sex Indicated** |
|---|---|---|---|
| 2 | CS3-2 Tube 1 | - | F |
| 4 | CS3-2 Tube 2 | - | F |
| 5 | CS3-2 Tube 3 | - | F |
| 6 | CS3-2 Tube 4 | + | M |
| 7 | CS3-2 Tube 5 | - | F |
| 8 | CS3-2 Tube 6 | + | M |
| 9 | CS3-2 Tube 7 | - | F |
| 10 | CS3-2 Tube 8 | - | F |
| 11 | CS3-2 Tube 9 | - | F |
| 12 | CS3-2 Tube 10 | - | F |
| 13 | CS3-2 Tube 11 | - | F |
| 14 | CS3-2 Tube 12 | - | F |
| 15 | Male Control | ± | M |
| 16 | Blank | - | na |
| 17 | Female Control | - | F |
| 18 | No DNA | - | na |

Finally, both the ammonia lysis and complement lysis procedures may be carried out in solution, as described above, or may be carried out using negative selection non-rare cell binding immobilization media, such as LEUKOSORB A and LEUKOSORB B (Pall Corporation). As described more fully in the Twitty, et al. patent application, LEUKOSORB A and LEUKOSORB B have binding affinities primarily for nucleated cells, but not mature enucleated erythroid cells. Thus, the complement lysing step, describe above, will first be applied to rid the matrix of bound unwanted cells, then the ammonium lysis step will be carried out on an affinity medium having a binding affinity for nucleated cell populations. The wanted cells are then removed from the matrix by increasing the salt concentration or with proteases.

The adjunct use of adsorption-filtration affinity matrices as solid phase supports for ammonium lysis and complement lysis, entails layering a CFS-separated fraction onto a fibrous adsorption-filtration filter medium having a nominal pore size of about 8 microns and being capable of 40-80% leukocyte immobilization, with a 70-80% post-wash leukocyte retention rate, and which is extremely hydrophilic, being capable of wetting with solutions having surface tensions of up to 85-90 dynes, which has a hold up volume of 40-70µl/cm² for a single layer of adsorption-filtration filter medium and which is characterized by low to medium protein binding. The preferred adsorption-filtration filter medium and which is characterized by low to medium protein binding. The preferred adsorption-filtration separation filter medium is that sold by Pall Corporation under the trademarks "LEUKOSORB" TYPES A and B or that described in U.S. Patent Nos. 4,923,620, 4,925,572, or European Patent No. 313348.

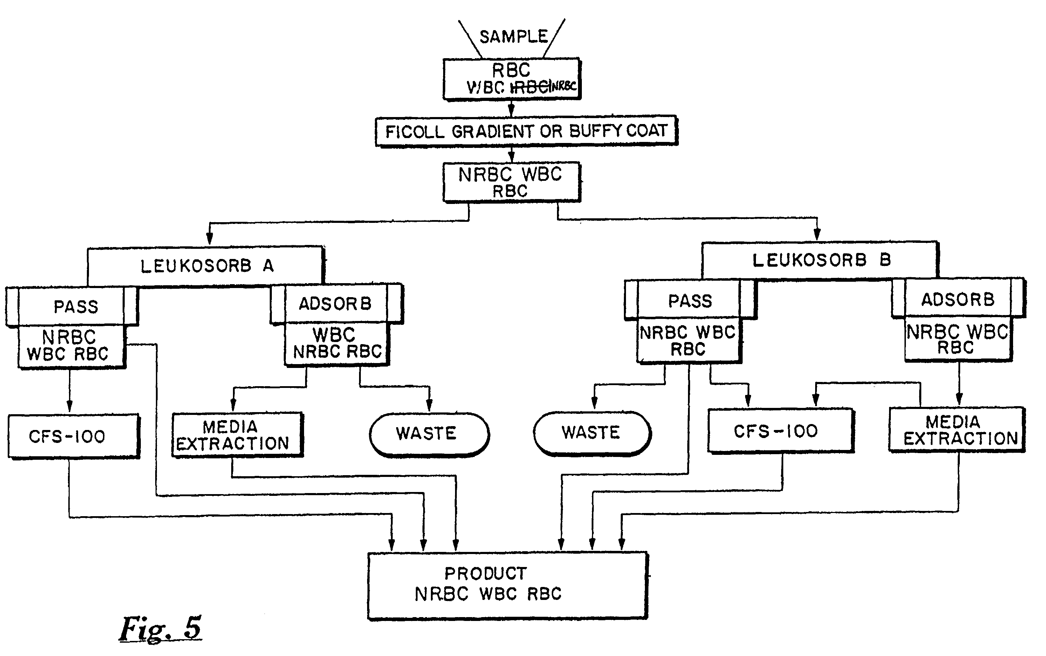

Turning now to Figures 5-8, there is a disclosed the use of at least one leukocyte-depleting matrix which preferentially captures leukocytes by at least one of adsorption, filtration and affinity as described in U.S. Patent No. 4,925,572 or PCT Publication No. WO 94/17209 . The preferable leukocyte-depleting matrices are "LEUKOSORB A" and "LEUKOSORB B" both manufactured and sold by Pall Corporation. For purposes of illustration only, the leukocyte-depleting matrices depicted in Figures 5-8 are identified by LEUKOSORB A and LEUKOSORB B, but any type of adsorption, filtration or affinity matrix which functions to preferentially pass nucleated red blood cells or white blood cells and red blood cells in a manner as described in Figures 5-8 is contemplated as being useful.

In Figure 5-8, the major fraction which passes through either the LEUKOSORB A or the LEUKOSORB B is identified by large capitol letters while the minor fraction passing and being adsorbed is indicated by smaller capital letters.

Figure 5 illustrates a first embodiment of the adsorption-filtration process in accordance with the present invention. A whole blood sample containing erythrocytes (RBC), leukocytes (WBC) and nucleated erythrocytes (NRBC) is layered onto a Ficoll gradient as described earlier. Density gradient centrifugation on the Ficoll gradient separates a major fraction containing NRBC and WBC with few RBC components. The NRBC/WBC fraction is passed either over a LEUKOSORB A 110 or a LEUKOSORB B adsorption-filtration medium. Passing the NRBC/WBC fraction 104 over the LEUKOSORB A in step passes a fraction which includes a major fraction of NRBC and an accompanying minor fraction of WBC and RBC, while a major fraction of WBC and an accompanying minor fraction of NRBC and RBC is adsorbed on the LEUKOSORB A. Alternatively, in step the NRBC/WBC fraction may be passed over a LEUKOSORB B medium, and a fraction of NRBC, WBC and RBC is passed, while a major fraction of NRBC and WBC, with an accompanying minor fraction of RBC is adsorbed.

The NRBC fraction from step is then passed through the CFS 60, described above, to further concentrate the desired rare cell population product. Further, the adsorbed fraction may be extracted by desorbing with an extraction medium appropriate for the adsorbed cell population to yield the desired cell population product.

Alternatively, if step is followed, the fraction passing through the LEUKOSORB is either discarded as waste or is concentrated by CFS 60, as described above. The adsorbed fraction may be desorbed to yield the desired cell population product, or concentrated by CFS 60, as described above, to yield the desired cell population product.

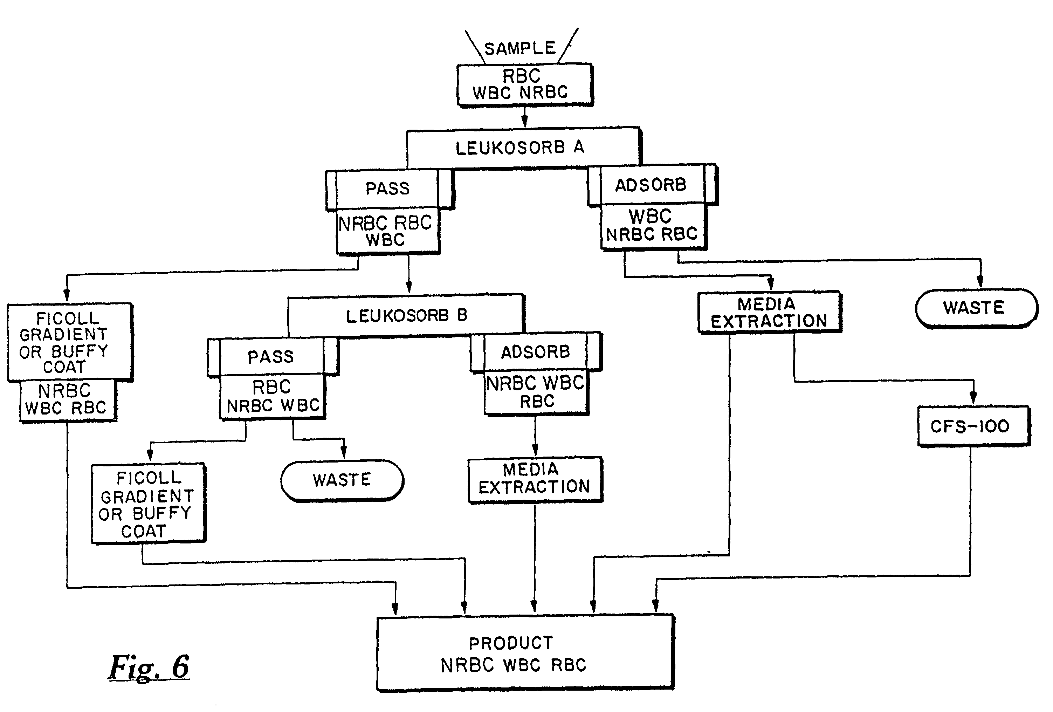

Figures 6-8, set forth a second embodiment of the adsorption-filtration process, a third embodiment of the adsorption-filtration process, and a fourth embodiment of the adsorption-filtration process, respectively. In each of embodiment, the basic process steps are identical, except that the order of performing the process steps has been altered between the embodiments as illustrated in the Figures. For example, in Figure 6, the sample is placed onto a LEUKOSORB A medium, then passed either to a Ficoll gradient for density gradient centrifugation and separation of the desired cell population, or the fraction passing is placed onto a LEUKOSORB B medium, and the fraction passing is subject to density gradient centrifugation to yield the product. The adsorbed fraction on the LEUKOSORB B medium, is extracted to yield the desired product. The fraction adsorbed on the LEUKOSORB A medium is extracted, and may be concentrated by CFS 60, to yield the desired cell population.

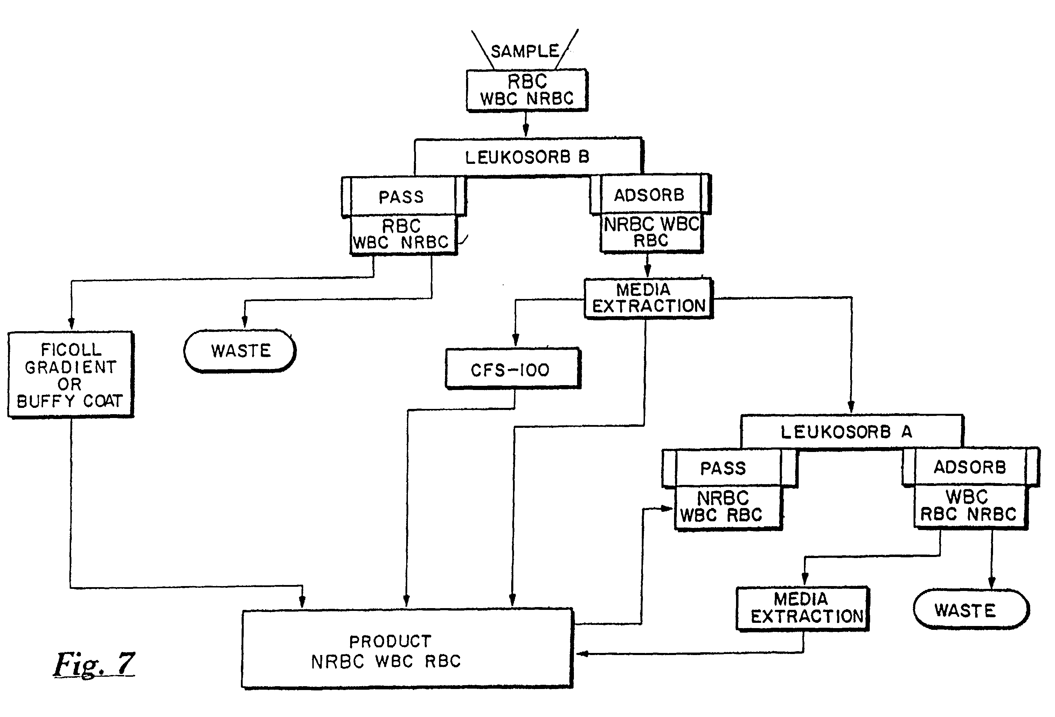

In Figure 7, the sample is placed onto a LEUKOSORB B medium, the fraction passed is subjected to density gradient centrifugation or is waste, while the adsorbed fraction is extracted and subjected to CFS 60 to yield the product, or the extracted fraction is passed onto LEUKOSORB A medium for further adsorption or filtration to either positively or negatively select the desired product.

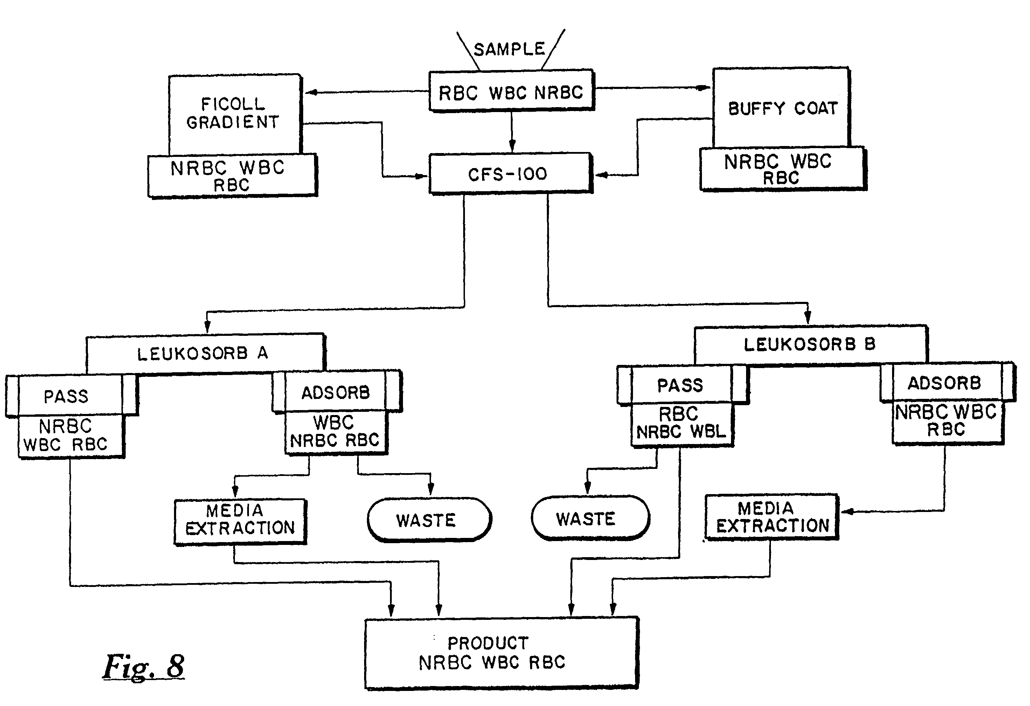

Finally, in Figure 8, as illustrated in the Example set forth above, the sample either first subjected to CFS 60, or is subjected to density gradient centrifugation followed by CFS 60. The concentrated sample from CFS 60, is then passed onto either LEUKOSORB A medium 110 and LEUKOSORB B medium, each either positively (passing) or negatively (adsorbing) the desired cell population.

It has been found that enrichment of rare fetal nucleated red cells from a maternal whole blood sample using Ficoll/Hypaque-1119 followed by charge flow separation (as illustrated in Figure 8) yielded rare cell populations at a level between 2-7%. By using the subsequent steps of ammonium lysis followed by complement lysis, the system and method of the present invention further increased rare cell population yield to a level up to about 17%. These levels of enrichment represent substantial and significant advances in recovery of rare cell populations from the peripheral blood circulation and achieve, for the first time, sufficient numbers of separated rare cells to make clinical diagnosis possible and meaningful.

## Claims

1. A method for recovering a desired cell population selected from the group consisting of nucleated cells, erythroid progenitor cells and fetal nucleated erythrocytes from a whole blood sample, comprising the steps of:
a) depleting a major fraction of unwanted cells from the whole blood sample; thereby concentrating a desired cell population;
b) passing the concentrated desired cell population through a counterflow stabilized charge-flow separator apparatus, in which the mobility of each cellular component in the blood sample in the presence of an applied electrical field is opposed by a buffer counterflow, thereby concentrating the desired cell population from other cellular blood components;
c) retrieving at least one fraction eluting from the charge-flow separator apparatus having a concentrated desired cell population; and
d) recovering an enriched desired cell population for analysis.

2. The method of Claim 1, wherein said step of depleting unwanted blood cells further comprises the step of ammonium lysis under conditions which lyse a substantial quantity of enucleate erythrocytes and without lysing substantial quantities of the desired cell population.

3. The method of Claim 2, wherein said step of depleting unwanted blood cells further comprises the step of complement lysis after the step of ammonium lysis under conditions which lyse a substantial quantity of lymphoid and myeloid cells and without lysing substantial quantities of the desired cell population.

4. The method of Claim 2, wherein said step of depleting unwanted blood cells further comprises the step of adsorbing the desired cell population onto an solid adsorption matrix having a greater binding affinity for the desired cell population than for the unwanted cell populations in the whole blood sample.

5. The method of Claim 2, wherein said step of depleting unwanted blood cells further comprises the step of adsorbing the unwanted cell populations onto a solid-phase adsorption matrix having a greater binding affinity for the unwanted cell populations in the whole blood sample than for the desired cell population.

6. A method according to claim 1 for enriching a whole blood sample for desired cell populations within the whole blood sample, comprising the steps of:
a) density gradient centrifugation of the whole blood sample in the presence of a Ficoll gradient having a density of at least about 1.119 g/ml at less than or equal to about 500 g;
b) recovering a desired fraction from the density gradient centrifugation and passing the desired fraction through a, counterflow stabilized charge-flow separator apparatus, in which the mobility of each cellular component in the blood sample in the presence of an applied electrical field is opposed by a buffer counterflow, thereby concentrating desired fraction;
c) retrieving at least one fraction eluting from the charge-flow separator apparatus having a concentrated desired fraction from step (b);
d) depleting unwanted cells from the concentrated desired fraction; and
e) recovering an enriched desired cell population for analysis.

7. The method of Claim 6 further comprising the step of obtaining a separated cell population of nucleated blood cells from the charge-flow separator apparatus and passing the separated cell population sample containing nucleated blood cells through a leukocyte depleting affinity matrix.

8. The method of Claim 6 further comprising the step of obtaining a separated cell population of nucleated blood cells from the charge-flow separator apparatus and passing the separated cell population sample containing nucleated blood cells through a leukocyte depleting adsorption-filtration matrix.

9. The method of Claim 6, wherein said step of depleting unwanted cells from the concentrated desired fraction further comprises the step of ammonium lysis under conditions which lyse a substantial quantity of enucleate erythrocytes and without lysing substantial quantities of the desired cell population.

10. The method of Claim 9, wherein said step of depleting unwanted cells from the concentrated desired fraction further comprises the step of complement lysis after the step of ammonium lysis under conditions which lyse a substantial quantity of lymphoid and myeloid cells and without lysing substantial quantities of the desired cell population.

11. A method according to any one of the preceding claims which further comprises the step of culturing the thus recovered enriched desired cell population.

## Patentansprüche

1. Verfahren zur Gewinnung einer gewünschten Zellpopulation, ausgewählt aus der Gruppe, bestehend aus kernhaltigen Zellen, erythroiden Vorläuferzellen und fetalen kernhaltigen Erythrozyten, aus einer Vollblutprobe, umfassend die Schritte:
a) Abreichern einer Hauptfraktion unerwünschter Zellen aus der Vollblutprobe, wodurch eine gewünschte Zellpopulation konzentriert wird,
b) Leiten der konzentrierten gewünschten Zellpopulation durch eine durch Gegenstrom stabilisierte Ladungsfluß-Trennvorrichtung, in der der Mobilität jeder Zellkomponente in der Blutprobe in der Gegenwart eines applizierten elektrischen Feldes durch einen Puffergegenstrom entgegengewirkt wird, wodurch die gewünschte Zellpopulation aus anderen Zellblutkomponenten konzentriert wird,
c) Wiedergewinnen mindestens einer Fraktion mit einer konzentrierten gewünschten Zellpopulation, die aus der Ladungsfluß-Trennvorrichtung eluiert und
d) Gewinnen einer angereicherten gewünschten Zellpopulation zur Analyse.

2. Verfahren nach Anspruch 1, wobei der Schritt des Abreicherns ungewünschter Blutzellen außerdem umfasst den Schritt der Ammoniumlyse unter Bedingungen, bei denen eine wesentliche Menge der enukleierten Erythrozyten lysiert werden, ohne das wesentliche Mengen der gewünschten Zellpopulation lysiert werden.

3. Verfahren nach Anspruch 2, wobei der Schritt des Abreicherns ungewünschter Blutzellen außerdem umfasst den Schritt der Komplementlyse nach dem Schritt der Ammoniumlyse unter Bedingungen, bei denen eine wesentliche Menge an lymphoiden und myeloiden Zellen lysiert wird, ohne das wesentliche Mengen der gewünschten Zellpopulation lysiert werden.

4. Verfahren nach Anspruch 2, wobei der Schritt des Abreicherns ungewünschter Blutzellen außerdem umfasst den Schritt des Adsorbierens der gewünschten Zellpopulation an eine feste Adsorptionsmatrix mit einer größeren Bindungsaffinität für die gewünschte Zellpopulation als für die ungewünschten Zellpopulationen in der Vollblutprobe.

5. Verfahren nach Anspruch 2, wobei der Schritt des Abreicherns ungewünschter Blutzellen außerdem umfasst den Schritt des Adsorbierens der unerwünschten Zellpopulation an eine Festphasenadsorptionsmatrix mit einer größeren Bindungsaffinität für die ungewünschten Zellpopulationen in der Vollblutprobe als für die gewünschte Zellpopulation.

6. Verfahren nach Anspruch 1 zur Anreicherung einer Vollblutprobe für gewünschte Zellpopulationen in der Vollblutprobe, umfassend die Schritte
a) Dichtegradienten-Zentrifugierung der Vollblutprobe in der Gegenwart eines Ficoll-Gradienten mit einer Dichte von mindestens 1,119 g/ml bei weniger als oder gleich ungefähr 500 g,
b) Gewinnen einer Fraktion aus der Dichtegradienten-Zentrifugierung und Leiten der gewünschten Fraktion durch eine Ladungsfluß-Trennvorrichtung, in der der Mobilität jeder Zellkomponente in der Blutprobe in der Gegenwart eines applizierten elektrischen Feldes durch einen Puffergegenstrom entgegengewirkt wird, wodurch die gewünschte Fraktion konzentriert wird,
c) Gewinnen mindestens einer Fraktion mit einer konzentrierten gewünschten Fraktion aus Schritt (b), die von der Ladungsfluß-Trennvorrichtung eluiert,
d) Abreichern unerwünschter Zellen aus der konzentrierten gewünschten Fraktion und
e) Gewinnen einer angereicherten gewünschten Zellpopulation zur Analyse.

7. Verfahren nach Anspruch 6, außerdem umfassend die Schritte des Erhaltens einer getrennten Zellpopulation aus kernhaltigen Blutzellen von der Ladungsfluß-Trennvorrichtung und Leiten der getrennten Zellpopulationsprobe, die kernhaltige Blutzellen enthält, durch eine Leukozyten abreichernde Affinitätsmatrix.

8. Verfahren nach Anspruch 6, außerdem umfassend den Schritt des Erhaltens einer getrennten Zellpopulation kernhaltiger Blutzellen aus der Ladungsfluß-Trennvorrichtung und Leiten der getrennten Zellpopulationsprobe, die kernhaltige Blutzellen enthält, durch eine Leukozyten abreichernde Adsorptionsfiltrierungsmatrix.

9. Verfahren nach Anspruch 6, wobei der Schritt des Abreicherns unerwünschter Zellen aus der konzentrierten gewünschten Fraktion außerdem den Schritt der Ammoniumlyse umfasst unter Bedingungen, bei denen eine wesentliche Menge enukleierter Erythrozyten lysiert wird, ohne das wesentliche Mengen der gewünschten Zellpopulation lysiert werden.

10. Verfahren nach Anspruch 9, wobei der Schritt des Abreicherns unerwünschter Zellen aus der konzentrierten gewünschten Fraktion außerdem den Schritt der Komplementlyse nach dem Schritt der Ammoniumlyse umfasst unter Bedingungen, bei denen eine wesentliche Menge an lymphoiden und myeoliden Zellen lysiert wird, ohne das wesentliche Mengen der gewünschten Zellpopulation lysiert werden.

11. Verfahren nach einem der vorhergehenden Ansprüche, das außerdem den Schritt des Kultivierens der so gewonnen angereicherten gewünschten Zellpopulation umfasst.

## Revendications

1. Procédé pour l'obtention d'une population cellulaire désirée sélectionnée dans le groupe consistant en des cellules nucléées, des cellules souches érythroïdes et des érythrocytes foetaux nucléés à partir d'un prélèvement de sang complet, comportant les étapes de :
a) épuisement d'une fraction principale de cellules non désirées à partir du prélèvement de sang complet, concentrant de ce fait une population cellulaire désirée ;
b) passage de la population cellulaire désirée concentrée à travers un séparateur de charge-flux stabilisé à contre-courant, dans lequel la mobilité de chaque composant cellulaire dans le prélèvement sanguin en présence d'un champ électrique appliqué est opposée par un contre-courant tampon, concentrant de ce fait la population cellulaire désirée à partir d'autres composants cellulaires du sang ;
c) extraction d'au moins une fraction éluant du séparateur de charge-flux ayant une population cellulaire désirée concentrée ; et
d) récupération d'une population cellulaire désirée enrichie pour analyse.

2. Procédé de la revendication 1, dans lequel ladite étape d'épuisement des cellules sanguines non désirées comporte en outre une étape de lyse à l'ammonium dans des conditions qui lysent une quantité substantielle d'érythrocytes énucléés sans lyser de quantités substantielles de la population cellulaire désirée.

3. Procédé de la revendication 2, dans lequel ladite étape d'épuisement des cellules sanguines non désirées comporte en outre une étape de lyse complémentaire après l'étape de lyse à l'ammonium dans des conditions qui lysent une quantité substantielle de cellules lymphoides et myéloïdes sans lyser de quantités substantielles de la population cellulaire désirée.

4. Procédé de la revendication 2, dans lequel ladite étape d'épuisement des cellules sanguines non désirées comporte en outre une étape d'adsorption de la population cellulaire désirée sur une matrice d'adsorption solide ayant une affinité de liaison pour la population cellulaire désirée supérieure à l'affinité pour les populations cellulaires non désirées dans le prélèvement de sang complet.

5. Procédé de la revendication 2, dans lequel ladite étape d'épuisement des cellules sanguines non désirées comporte en outre une étape d'adsorption des populations cellulaires non désirées sur une matrice d'adsorption à phase solide ayant une affinité de liaison pour les populations cellulaires non désirées dans le prélèvement de sang complet supérieure à l'affinité pour la population cellulaire désirée.

6. Procédé selon la revendication 1 pour l'enrichissement d'un prélèvement de sang complet en populations cellulaires désirées au sein du prélèvement de sang complet, comportant les étapes de :
a) centrifugation en gradient de densité du prélèvement de sang complet en présence d'un gradient de Ficoll ayant une densité d'au moins environ 1,119 g/ml à moins de ou égale à environ 500 g ;
b) récupération d'une fraction désirée issue de la centrifugation en gradient de densité et passage de la fraction désirée à travers un séparateur de charge-flux stabilisé à contre-courant, dans lequel la mobilité de chaque composant cellulaire dans le prélèvement sanguin en présence d'un champ électrique appliqué est opposée par un contre-courant tampon, concentrant de ce fait la fraction désirée ;
c) extraction d'au moins une fraction éluant du séparateur de charge-flux ayant une fraction désirée concentrée de l'étape (b) ;
d) épuisement des cellules non désirées à partir de la fraction désirée concentrée ; et
e) récupération d'une population cellulaire désirée enrichie pour analyse.

7. Procédé de la revendication 6 comportant en outre une étape d'obtention d'une population cellulaire distincte de cellules sanguines nucléées à partir du séparateur de charge-flux et de passage du prélèvement de population cellulaire distincte contenant des cellules sanguines nucléées à travers une matrice d'affinité d'épuisement des leucocytes.

8. Procédé de la revendication 6 comportant en outre une étape d'obtention d'une population cellulaire distincte de cellules sanguines nucléées à partir du séparateur de charge-flux et de passage du prélèvement de population cellulaire distincte contenant des cellules sanguines nucléées à travers une matrice d'adsorption-filtration d'épuisement des leucocytes.

9. Procédé de la revendication 6, dans lequel ladite étape d'épuisement des cellules non désirées à partir de la fraction désirée concentrée comporte en outre une étape de lyse à l'ammonium dans des conditions qui lysent une quantité substantielle d'érythrocytes énucléés sans lyser de quantités substantielles de la population cellulaire désirée.

10. Procédé de la revendication 9, dans lequel ladite étape d'épuisement des cellules non désirées à partir de la fraction désirée concentrée comporte en outre une étape de lyse complémentaire après l'étape de lyse à l'ammonium dans des conditions qui lysent une quantité substantielle de cellules lymphoïdes et myéloïdes sans lyser de quantités substantielles de la population cellulaire désirée.

11. Procédé selon l'une quelconque des revendications précédentes qui comporte en outre une étape de mise en culture de la population cellulaire désirée enrichie ainsi récupérée.
